# EUROPÄISCHE PATENTSCHRIFT

(11) **EP 2 420 195 B1**
(45) Veröffentlichungstag und Bekanntmachung des Hinweises auf die Patenterteilung: **19.07.2017**
(21) Anmeldenummer: 11177980.7
(22) Anmeldetag: 18.08.2011
(51) Int. Cl.: A61B 18/02, F16L 37/56, A61M 39/10

(54) **Vorrichtung zum fluidführenden Anschluss wenigstens einer Applikationssonde an einen Versorgungsschlauchsatz und Handgriff für ein Chirurgieinstrument**
Device for the fluid-conveying connection of at least one application probe with a supply tubing set, and handle for a surgical instrument
Dispositif de raccordement transportant des fluides d'au moins une sonde d'application sur un ensemble de tuyaux flexibles de distribution et poignée pour un instrument chirurgical

(30) Priorität: 18.08.2010 DE 102010037026
(43) Veröffentlichungstag der Anmeldung: 22.02.2012
(73) Patentinhaber: Erbe Elektromedizin GmbH, 72072 Tübingen (DE)
(72) Erfinder: Amann, Markus, 72070 Tübingen (DE); Besch, Hansjörg, 72810 Gomaringen (DE)
(74) Vertreter: Rüger, Barthelt & Abel

(56) Entgegenhaltungen:
- WO-A2-2009/090647
- GB-A- 1 111 757
- US-A- 3 272 203
- US-A- 3 536 075
- US-A- 5 573 532

## Beschreibung

Die Erfindung betrifft eine Vorrichtung zum fluidführenden Anschluss wenigstens einer Applikationssonde, mit wenigstens zwei koaxial geführten Applikationsieltungselementen, an einen Versorgungsschlauchsatz, mit wenigstens zwei parallel geführten Versorgungsleitungselementen, in einem Griffelement eines Chirurgie- und insbesondere Kryochirurgieinstrumentes, derart, dass im angeschlossenen Zustand jedes Versorgungsleitungselement mit wenigstens einem zugehörigen Applikationsleitungselement in Fluidverbindung steht.

Darüber hinaus betrifft vorliegende Erfindung auch einen Handgriff für ein ChirurgieInstrument, insbesondere Kryochirurgieinstrument, mit einem Griffelement, einem Versorgungsschlauchsatz, mit wenigstens zwei parallel geführten Versorgungsleitungselementen, und wenigstens einer Applikationssonde, mit wenigstens zwei koaxial geführten Applikationsleitungselementen, wobei jedes Versorgungsleitungselement im Griffelement mit einem zugehörigen Applikationsleitungselement verbindbar oder verbunden ist.

Derartige Handgriffe und Anschlussvorrichtungen innerhalb solcher Handgriffe sind aus dem Stand der Technik bekannt. Meist werden die Handgriffe mit einem Versorgungsschlauchsatz und einer Applikationssonde bestückt, die über den Versorgungsschlauchsatz mit einem Applikationsfluid gespeist wird. Die erwähnten Anschlussvorrichtungen dienen dem fluidführenden Anschluss des Schlauchsatzes an die Applikationssonde, um beispielsweise über den Schlauchsatz und die darin ausgebildeten Versorgungsleitungselemente ein Applikationsfluid der Applikationssonde zu- und von dieser abzuführen. Dazu wird das Fluid über wenigstens ein Versorgungsleitungselement dem Griffelement und von dort der Applikationssonde zugeführt, wo es über ein Im Außenumfang der Applikationssonde geführtes Applikationsleitungselement zu einem Applikationssondenkopf fließt. Von dort wird es dann über ein koaxial innengeführtes Applikationsleitungselement zurückgeführt und im Griffelement in ein rückleitendes Versorgungsleitungselement übergeleitet, über das es zur Versorgungseinheit zurück fließt.

Ein wesentlicher Punkt bei obigen Vorrichtungen ist die Verbindung zwischen den parallel geführten Versorgungsleitungselementen bzw. dem Versorgungsschlauchsatz und den koaxial geführten Applikationsleitungselementen bzw. der Applikationssonde.

Zum Anschluss bzw. zur Umleitung zwischen paralleler und koaxialer Leitungsführung ist aus dem Stand der Technik eine Mehrzahl an Vorrichtungen bekannt, die allesamt einen sehr komplexen, vor allem aber empfindlichen und sehr raumgreifenden Aufbau aufweisen.

In Fachkreisen besteht ein Bedarf an Handgriffen und somit auch der Bedarf an Anschlussvorrichtungen, die ein wechselseitiges Beschicken der Applikationssonden erlauben. Darunter wird verstanden, dass die Handgriffe bzw. Chirurgieinstrumente beispielsweise nicht nur als "normale" Kryochirurgieinstrumente sondern auch als Defreeze-Geräte verwendet werden können, bei denen der Zulauf des noch warmen Fluids außen und der Rücklauf des abgekühlten Fluids innen erfolgen. Bei diesem "umgekehrten" Anwendungsfall muss der Betrieb der Applikationssonde in umgekehrter Fluidflussrichtung möglich sein.

Da darüber hinaus aufgrund der sehr hohen Kosten im Chirurgieinstrumentenbereich der Trend zu modular und mit geringer Teilevielfalt aufgebauten Instrumente geht, besteht ein Bedarf an Handgriffen bzw. nach Chirurgieinstrumenten, bei denen beispielsweise der Handgriff auf einfache Weise mit unterschiedlichen Applikationssonden bestückt werden kann, ohne dass es zu aufwändigen Umbaumaßnahmen am Gerät kommen muss.

Diese Aufgaben werden durch den bekannten Stand der Technik nur unzureichend gelöst.

Aufgabe der vorliegenden Erfindung ist es folglich, ein Chirurgieinstrument bzw. dafür geeignete Vorrichtungen der vorgenannten Art anzubieten, die einen einfachen, kostengünstigen und verlässlichen Anschluss wenigstens eines, vorzugsweise aber mehrerer unterschiedlicher Applikationssonden an einen Versorgungsschlauchsatz ermöglichen.

Diese Aufgabe wird durch einen Handgriff für ein Chirurgieinstrument gemäß Anspruch 1 gelöst. Ein wesentlicher Punkt der Erfindung ist die Ausbildung eines Fluidweichenelementes im Griffelement, wobei dieses Fluidweichenelement Anschlussbereiche sowohl für die Applikationsleitungselemente als auch die Versorgungsleitungselemente anbietet und diese jeweiligen Leitungselemente über integral innerhalb des Fluidweichenelementes ausgebildete Übergangselemente in Fluidverbindung stellt. Auf diese Weise kann das Fluidweichenelement sehr kompakt und kostengünstig hergestellt werden, wobei es sehr raumsparende geometrische Abmessungen aufweisen kann und so auch in sehr kleine chirurgische Instrumente einbaubar ist.

Über das erfindungsgemäße Fluidweichenelement kann so also beispielsweise ein Versorgungsschlauchsatz mit zwei oder mehr Versorgungsleitungselementen am Griffelement bzw. einem darin angeordneten Fluidweichenelement angeordnet werden, wobei die jeweiligen Versorgungsleitungselemente dann sehr einfach, platzsparend und kostengünstig über die integral ausgebildeten Übergangselemente mit einer am Applikationsleitungsanschlussbereich angeordneten Applikationssonde in Fluidverbindung bringbar sind, wobei insbesondere der Leitungsverzug zwischen den parallel geführten Versorgungsleitungselementen und den koaxial geführten Applikationsleitungselementen zuverlässig und platzsparend gelöst ist.

Vorzugsweise ist das Fluidweichenelement als ein einstückiges Drehteil, Frästeil oder dergleichen hergestelltes Element ausgebildet. Unter einstückiger Ausbildung wird hier verstanden, dass wenigstens entsprechende Aufnahmebereiche für den Applikationsleitungsanschlussbereich, den Versorgungsleitungsanschlussbereich und die zuvor beschriebenen Übergangselemente einstückig im Fluidweichenelement ausgebildet sind. So ist es also beispielsweise möglich, entsprechende Aufnahmebereiche am Fluidweichenelement auszubilden, in die dann entsprechende Dichtungselemente zum fluiddichten Anschluss der jeweiligen Versorgungs- oder Applikationsleitungselemente eingebracht werden können.

Vorzugsweise sind insbesondere die Überleitungselemente im Innenraum des Fluidweichenelementes geführt, ausgefräst, gegossen oder dergleichen hergestellt.

Vorzugsweise ist das Fluidweichenelement wenigstens teilweise als ein Zylinderelement oder dergleichen langgestreckter Volumenkörper ausgebildet. Gerade eine solche kompakte Bauweise erlaubt den einfachen und sicheren Einbau in ein Griffelement eines Chirurgieinstrumentes.

Vorzugsweise sind der Versorgungsleitungsanschlussbereich und/oder der Applikationsleitungsanschlussbereich an Stirnseiten oder sich dergleichen gegenüberliegenden Seiten des Zylinderelementes oder des dergleichen langgestreckten Volumenkörpers angeordnet. Auf diese Weise ist der Einbau des Fluidweichenelementes in den vorzugsweise langgestreckt ausgebildeten Griffelementen von Chirurgieinstrumenten sehr einfach möglich.

Vorzugsweise sind der Versorgungsleitungsanschlussbereich und/oder der Appllkationsleitungsanschlussbereich in einem integral im Fluidweichenelement ausgebildeten Aufnahmebereich derart angeordnet, dass sie nicht über die Außengeometrie der Fluidweiche hervorstehen. Wie zuvor schon erwähnt, bilden der oder die Aufnahmebereiche für die jeweiligen Anschlussbereiche am Fluidweichenelement geeignete Freiräume, um die jeweiligen Leitungselemente, unter Umständen aber auch den Schlauchsatz bzw. die Applikationssonde wenigstens teilweise im Fluidwelchenelement aufzunehmen, wobei die zur fluiddichten Verbindung der jeweiligen Elemente nötigen Dicht- evtl. aber auch weitere Klemm- und dergleichen Elemente vorzugsweise vollständig in den jeweiligen Aufnahmebereichen aufgenommen sind.

Vorzugsweise weisen das Fluidweichenelement und insbesondere der Applikationsleitungsanschlussbereich einen Applikationssondenbereich derart auf, dass er die lösbare Aufnahme der Applikationssonde am Fluidweichenelement erlaubt. Der Applikationssondenaufnahmebereich ist also vorzugsweise so ausgebildet, dass die Applikationssonde mit einem komplementär ausgebildeten Applikationssondenformteil mit dem Applikationssondenaufnahmebereich in Wirkverbindung tritt und zwar derart, dass die Sonde richtig positioniert, vorzugsweise an der Vorrichtung oder einem benachbarten Griffelement oder dergleichen Element fixiert und darüber hinaus fluiddicht im Applikationsleitungsanschlussbereich angeschlossen ist. Der Applikationssondenaufnahmebereich kann also beispielsweise eine koaxial zum Applikationsleitungsanschlussbereich ausgebildeter Aufnahmebereich sein, in dem die Applikationssonde mit einem entsprechend ausgebildeten Applikationssondenformteil einführbar ist, wobei im eingeführten Zustand die Applikationsleitungselemente mit den Übergangselementen in Fluidverbindung treten und gleichzeitig die Applikationssonde an der Fluidweiche und/oder an einem zugeordneten Griffelement oder dergleichen Element fixiert ist.

Der Vorteil einer solchen Ausführungsform ist, dass auf sehr einfache Weise die Applikationssonde mit dem Versorgungsschlauchsatz verbunden werden kann, wobei insbesondere auch eine Mehrzahl unterschiedlich ausgebildeter Applikationssonden mit identisch ausgebildeten Applikationssondenformteilen zum Anschluss an den Applikationsanschlussbereich bzw. an den Applikationssondenaufnahmebereich mit dem Schlauchsatz verbunden werden können.

Vorzugsweise ist dabei der Applikationssondenaufnahmebereich integral im Fluidweichenelement ausgebildet und insbesondere derart, dass auf die Applikationssonde einwirkende mechanische Belastungen über das Fluidweichenelement abgetragen und von diesem insbesondere in das Griffelement weitergeleitet werden können.

Vorzugsweise weist der Applikationssondenaufnahmebereich wenigstens ein Arretierungsmittel auf, über das die Applikationssonde insbesondere werkzeuglos lösbar am Fluidweichenelement und/oder einem zugeordneten Griff oder dergleichen Element arretierbar ist. Ein solches Arretierungsmittel kann beispielsweise ein Bajonettverschlusselement sein, über das die Applikationssonde am Applikationssondenaufnahmebereich arretierbar ist. Hier sind sämtliche aus dem Stand der Technik bekannte Arretierungsmittel anwendbar und insbesondere solche Arretierungsmittel anwendbar, die die werkzeuglose und somit sehr schnelle Arretierung und De-Arretierung der Applikationssonde erlauben.

Vorzugsweise weist der Versorgungsleitungsanschlussbereich für jedes anzuschließende Versorgungsleitungselement jeweils ein komplementäres Versorgungsleitungsanschlusselement auf, über das das jeweilige Versorgungsleitungselement mit dem korrespondierenden Überleitungselement fluiddicht verbindbar ist, wobei die Versorgungsleitungsanschlusselemente und/oder die Versorgungsleitungselemente derart komplementär zueinander ausgebildet sind, dass wahlweise jedes Versorgungsleitungselement an jedes Versorgungsleitungsanschlusselement anschließbar ist. Auf diese Weise ist es also möglich, die Leitungsführung innerhalb des Griffelementes so zu verändern, dass beispielsweise ein als Kryochirurgie verwendetes Instrument auch als Defreeze-Instrument verwendbar ist. Wird in einem ersten Behandlungsfall ("Defreeze-Sonde") ein Applikationsfluid über die parallel geführten Versorgungsleitungselemente so der Applikationssonde zugeführt, dass das (noch warme) Applikationsfluid im außenseitig an der Applikationssonde angeordneten Applikationsleitungselement zum Applikationssondenkopf geführt und (abgekühlt) innenseitig wieder zurückgeführt wird, kann durch einen entsprechenden Wechsel der Fluidführung zwischen Versorgungsleitungsanschlusselementen und Versorgungsleitungselementen oder aber durch einen entsprechenden Wechsel im Anschlussbereich an der Versorgungseinheit eine entgegengesetzte Fluidführung erreicht werden, bei der das (noch warme) Applikationsfluid Im innenseitig angeordneten Applikationsleitungselement zum Applikationssondenkopf nach vorne geführt und (abgekühlt) im koaxial außenseitig geführten Applikationsleitungselement zurückgeführt wird. Ein solcher Austausch in der Fluidführung kann insbesondere durch ein entsprechend ausgebildetes Umpolelement erreicht werden, das im Schlauchsatz oder auch im Fluidweichenelement ausgebildet ist.

Vorzugsweise ist im Fluidweichenelement integral ein Filteraufnahmeraum zur Aufnahme eines Filterelementes derart ausgebildet, dass das Filterelement innerhalb wenigstens eines Fluidweges zwischen einem Versorgungsleitungselement und einem Applikationsleitungselement angeordnet ist. Auf diese Weise kann also beispielsweise innerhalb eines als Zulauf verwendeten Leitungselementes ein Filter angeordnet werden, um eine mögliche Verstopfung des nachgeschalteten Applikationssondenkopfes und dessen meist sehr viel dünnerer Leitungselemente zu verhindern. Vorzugsweise ist der Aufnahmeraum dabei so ausgebildet, dass er den Einsatz des Filters oder auch mehrerer Filter auch bei einer, wie zuvor beschrieben umgekehrten Leitungsführung erlaubt, so dass also das Filterelement sowohl in einem Zulaufelement als auch einem Rücklaufelement, das nach dem "Umschalten" ebenso ein Zulaufelement ist, angeordnet sein kann.

Die Erfindung betrifft, wie eingangs bereits erwähnt, neben der Anschlussvorrichtung auch einen Handgriff für ein Chirurgieinstrument der vorgenannten Art, das über eine solche Anschlussvorrichtung verfügt. Sämtliche zuvor genannten Spezifikationen dieser Vorrichtung gelten naturgemäß auch für einen solchen Handgriff, wobei hier aus Redundanzgründen auf eine explizite eigene Nennung verzichtet wird.

Weitere Ausführungsformen der Erfindung ergeben sich aus den Unteransprüchen.

Im Folgenden wird die Erfindung anhand von zwei Ausführungsformen beschrieben, die durch die beiliegenden Zeichnungen näher erläutert werden. Hierbei zeigen:
- Fig. 1: Eine schematische Darstellung einer Ausführungsform eines Chirurgieund insbesondere Kryochirurgiegerätes;
- Fig. 2 und 3: Querschnitte durch zwei Ausführungsformen eines Griffelementes des Chirurgiegerätes gemäß Fig. 1;
- Fig. 4: zwei isometrische Darstellungen einer Fluidweiche, wie sie in dem Griffelement gemäß den Fig. 2 und 3 verbaut ist;
- Fig. 5: einen Längsschnitt durch die Fluidweiche aus Fig. 4 gemäß der Schnittführung aus Fig. 6;
- Fig. 6: eine Ansicht der Fluidweiche gemäß der Sichtlinie aus Fig. 5;
- Fig. 7: einen Längsschnitt durch die Fluidweiche aus Fig. 4 gemäß der Schnittführung aus Fig. 8; und
- Fig. 8 und 9: Ansichten der Fluidweiche gemäß der Schnittführung aus Fig. 7.

Im Folgenden werden für gleiche und gleich wirkende Bauteile dieselben Bezugsziffern verwendet, wobei zur Unterscheidung bisweilen Hochindizes Anwendung finden.

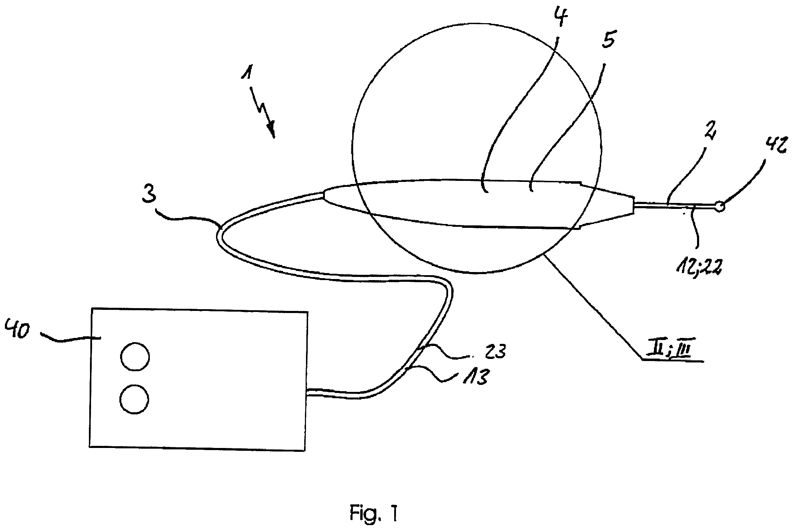

Fig. 1 zeigt eine Ausführungsform eines Chirurgieinstrumentes 1, wie es beispielsweise bei der Kryochirurgie Anwendung findet.

Dargestellt ist eine Versorgungseinheit 40, die einen Handgriff 5 über einen Versorgungsschlauchsatz 3 mit einem Applikationsfluid versorgt. Der Versorgungsschlauchsatz 3 umfasst dabei zwei Versorgungsleitungselemente 13, 23, von denen eines als zuleitendes Versorgungsleitungselement 13 und das andere als ableitendes Versorgungsleitungselement 23 ausgebildet ist, um das Applikationsfluid von der Versorgungseinheit 40 dem Handgriff 5 zu bzw. abzuleiten.

Der Handgriff 5 seinerseits umfasst ein Griffelement 4, das der Aufnahme des Schlauchsatzes 3 bzw. der Versorgungsleitungselemente 13, 23 und einer Applikationssonde 2 dient, die hier als Kryochirurgiesonde mit einer koaxialen Leitungsführung und koaxial geführten Applikationsleitungselementen 12, 22 ausgebildet ist.

Wie im Folgenden noch detailliert beschrieben, wird bei dem hier dargestellten Chirurgieinstrument also ein Applikationsfluid über die Versorgungseinheit 40 und ein zuleitendes Versorgungsleitungselement 13 des Schlauchsatzes 3 dem Griffelement 4 zugeführt und von dort, je nach Schaltzustand und angeschlossener Applikationssonde 2 über ein Applikationsleitungselement 12 einem Applikationssondenkopf 42 zugeführt und über ein zugeordnetes rückführendes Applikationsleitungselement 22 zurück zum Griffelement 4 und von dort über das abführende Versorgungsleitungselement 23 zu der Versorgungseinheit 40 geführt.

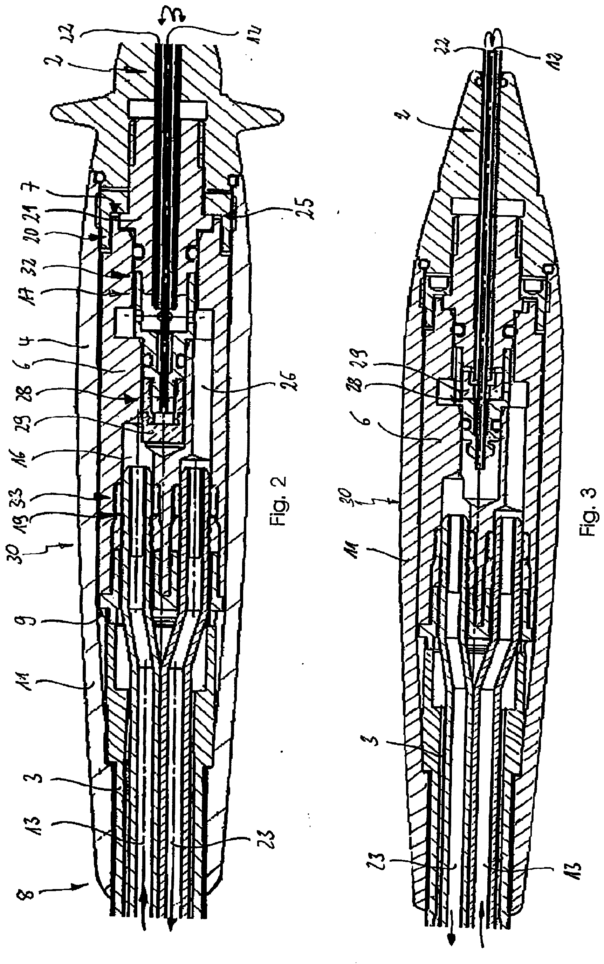

Wie in den Fig. 2 und 3 dargestellt, erfolgt dabei die Verbindung der Versorgungsleitungselemente 13, 23 mit den Applikationsleitungselementen 12, 22 über eine Anschlussvorrichtung 30, die als wesentliches Bauteil ein Fluidweichenelement 6 aufweist, das im Griffelement 4 angeordnet oder anordenbar ist und einen Applikationsleitungsanschlussbereich 32 aufweist zum Anschluss der Applikationsleitungselemente 12, 22 und einen Versorgungsleitungsanschlussbereich 33 aufweist zum Anschluss der Versorgungsleitungselemente 13, 23, Zudem weist das Fluidweichenelement 6 wenigstens zwei integral im Fluidweichenelement 6 ausgebildete Überleitungselemente 16, 26 auf, über die der Applikationsleitungsanschlussbereich 32 mit dem Versorgungsleitungsanschlussbereich 33, bzw. die jeweiligen dort angeordneten Applikations- bzw. Versorgungsleitungselemente 12, 22; 13, 23 in Fluidverbindung stehen.

Das Fluidweichenelement 6 ist dabei als eigenständiges Bauteil ausgebildet, so dass die Versorgungsleitungselemente 13, 23 und die Applikationsleitungselemente 12, 22 platzsparend und sicher daran anschließbar und so miteinander in Fluidverbindung bringbar sind.

Die Fig. 2 und 3 zeigen in einem Ausschnitt zwei Ausführungsformen eines Griffelementes 4 des Chirurgieinstrumentes 1, wie es in Fig. 1 dargestellt ist, wobei sich die beiden Ausführungsformen im Wesentlichen durch eine unterschiedliche Anordnung, insbesondere durch eine "Umpolung" der für den Zulauf bzw. den Ablauf verwendeten Versorgungsleitungselemente 13, 23 unterscheiden.

So zeigt Fig. 2 ein Griffelement 4 an dessen proximalem Ende 8 der Versorgungsschlauchssatz 3 mit seinen zwei Versorgungsleitungselementen 13, 23 angeordnet ist. Das Versorgungsleitungselement 13 dient dabei der Zuleitung eines Applikationsfluides, während das Versorgungsleitungselement 23 der Ableitung dient. Über das Fluidweichenelement 6 sind die beiden Versorgungsleftungselemente 13, 23 derart mit der Applikationssonde 2 bzw. deren Applikationsleitungselementen 12, 22 verbunden, dass das Applikationsfluid über das zuleitende Versorgungsleitungselement 13 in das innere Applikationsleitungselement 12 eingeleitet, von dort zum Applikationssondenkopf 42 (siehe Fig. 1) geleitet wird, dort expandiert und dabei abgekühlt und in das koaxial äußere Applikationsleitungselement 22 eingeleitet wird, darin zurückfließt und das Griffelement 4 über das Versorgungsleitungselement 23 verlässt.

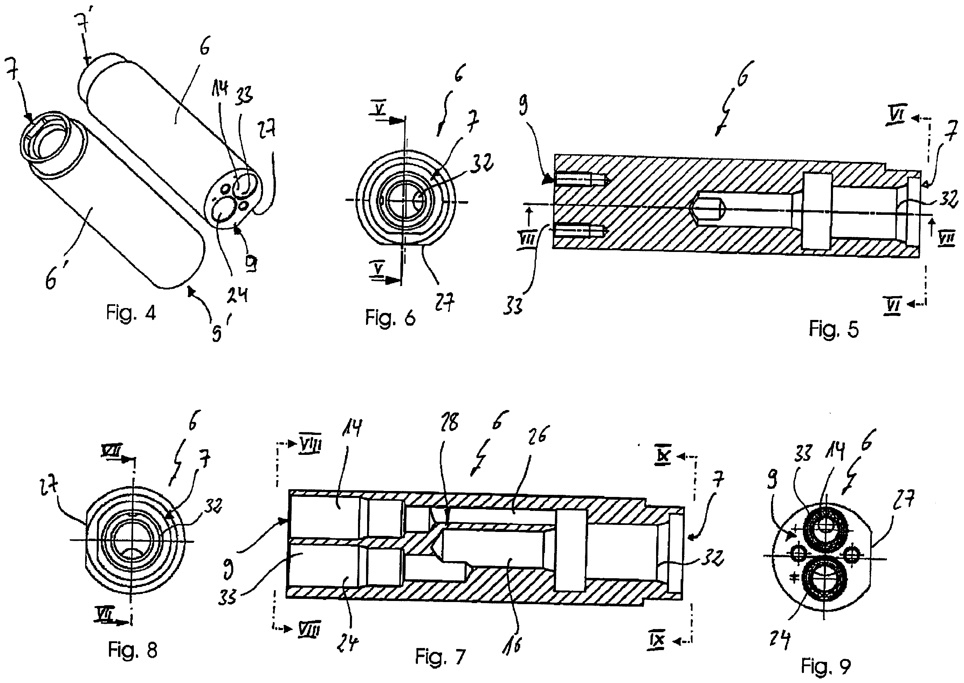

Die Verbindung der Leitungselemente 12, 13 bzw. 23, 22 erfolgt, über das erfindungsgemäße Fluidweichenelement 6, das hier, siehe insbesondere auch die Fig. 4 bis 9 als im Wesentlichen zylinderförmiges Element ausgebildet ist. Das Fluidweichenelement 6 weist an seinen beiden Stirnseiten 7 und 9 Anschlussbereiche, nämlich den Applikationsleitungsanschlussbereich 32 zum Anschluss der Applikationsleitungselemente 12, 22 und den Versorgungsleitungsanschlussbereich 33 zum Anschluss der Versorgungsleitungselemente 13, 23 auf. Der Versorgungsleitungsanschlussbereich 33 ist dabei erfindungsgemäß mit dem Applikationsleitungsanschlussbereich 32 über die integral im Fluidweichenelement 6 ausgebildeten Überleitungselemente 16, 26 verbunden, so dass das zugeführte Applikationsfluid zur Applikationssonde 2 und von dieser wieder abtransportiert werden kann.

In der Fluidverbindung zwischen dem zuleitenden Versorgungsleitungselement 13 und dem koaxial innengelegenen Applikationsleitungselement 12 ist ein Filteraufnahmeraum 28 angeordnet, in dem ein Filterelement 29 in die Fluidverbindung zwischen den beiden Leitungselementen 12, 13 angeordnet bzw. anordbar ist. Dieses Filterelement 29 filtert Verschmutzungen, die sich möglicherweise im zugeführten Applikationsfluid befinden heraus und verhindert eine Verstopfung der sehr dünnen Applikationsleitungselemente 12, 22. Wie Im Folgenden noch detailliert beschrieben, ist der Filteraufnahmeraum 28 so ausgebildet, dass er nicht nur die Anordnung des Filterelements 29 im zuleitenden Bereich, sondern auch im ableitenden Bereich, also in der Fluidverbindung zwischen dem Applikationsleitungselement 22 und dem zuführenden Versorgungsleitungselement 13 erlaubt (siehe Fig. 3). Wird das Filterelement 29, wie insbesondere in Fig. 3 dargestellt, dort verbaut, ist es möglich, das Chirurgieinstrument 1 Im "Umkehrbetrieb", also beispielsweise im Entfreeze-Betrieb zu betreiben, bei dem die Zu- und Ableitung des Applikationsfluides umgekehrt erfolgt.

Die Applikations- und Versorgungsleitungsanschlussbereiche 32, 33 sind bei dieser Ausführungsform so ausgeführt, dass sie über jeweils einen Aufnahmebereich 17 bzw. 19 verfügen, der derart im Fluidweichenelement 6 ausgebildet und angeordnet ist, dass der Versorgungsleitungsanschlussbereich 33 bzw. der Applikationsleitungsbereich 32 nicht über die Außengeometrie des Fluidweichenelementes 6 hervorstehen. Das bedeutet, dass die jeweilig zugeführten und abgeführten Leitungselemente 12, 13, 22, 23 innerhalb der integral ausgebildeten Aufnahmebereiche 17, 19 fluiddicht mit der Fluidweiche 6 verbunden und so in einer miteinander kommunizierenden Fluidverbindung stehen.

Auf das im Wesentlichen zylinderförmige Fluidweichenelement 6 ist ein Außenmantel 11 direkt aufgespritzt, was zu einem sehr einfach und kostengünstigen, darüber hinaus jedoch äußerst widerstandsfähigem Chirurgieinstrument führt.

Das einstückig ausgebildete Fluidweichenelement 6 umfasst an seinem einen Stirnseitenende 7 zudem einen Applikationssondenaufnahmebereich 20, über den die Applikationssonde 2 zusammen mit den dort ausgebildeten Applikationsleitungselementen 12, 22 am Fluidweichenelement 6 bzw. am Griffelement 4 befestigt und insbesondere arretiert werden kann. Die Applikationssonde 2 umfasst dazu ein Aufnahmeformteil 21, das komplementär zum Applikationsleitungsanschlussbereich 32 ausgebildet ist *und* zwar derart, dass die Applikationssonde 2 einfach und vor allem werkzeuglos am Griffelement 4 bzw. dem Fluidweichenelement 6 befestigt werden kann. Dazu ist am Aufnahmeformteil 21 ein Arretierungsmittel 25 vorgesehen, über das die Applikationssonde 2 lösbar am Fluidweichenelement 6 bzw. Griffelement 4 festgelegt werden kann. Hier sind sämtliche aus dem Stand der Technik bekannte Arretierungsmittel, beispielsweise Bajonettverschlüsse oder Rastelemente anwendbar.

In vorteilhafter Weise kann also bei der hier dargestellten Ausführungsform das Griffelement 4 mit unterschiedlichen Applikationssonden 2 versehen werden, wobei der Austausch auf sehr einfache und vor allem schnelle Weise erfolgen kann.

Wie bereits erwähnt, zeigt Fig. 3 eine zur Fig. 2 ähnliche Ausführungsform, wobei *hier* eine "umgepolte" Leitungsführung der Versorgungsleitungselemente 13, 23 vorgesehen ist. Im Gegensatz zu dem zuvor beschriebenen Griffelement wird bei der Ausführungsform gemäß Fig. 3 das Applikationsfluid über (ein anderes) zuleitendes Versorgungselement 13 dem koaxial äußeren Applikationsleitungselement 22 zugeführt, dort dem Applikationssondenkopf 42 (siehe Fig. 1) zugeleitet, dann über das koaxial innen gelegene Applikationsleitungselement 12 rückgeführt und über das ableitende Versorgungsleitungselement 23 abgeleitet. Um bei dieser sogenannten Defreeze-Technik verwendeten Leitungsführung ein Verstopfen der Applikationssonde 2 zu verhindern, ist erfindungsgemäß das Filterelement 29 nun an einer anderen zuleitenden Fluidver-bindung zwischen dem Versorgungsleitungselement 13 und dem Applikationsleitungs-element 22 angeordnet. Ansonsten entspricht die hier dargestellte Ausführungsform der Ausführungsform zu Flg. 2.

Die Fig. 4 bis 9 zeigen die zuvor beschrieben Fluidweiche 6 in perspektivischer Darstellung (Fig. 4) bzw. in Längsschnitten (Fig. 5 und Fig. 7) sowie Stirnseitenansichten (Fig. 6, Fig. 8, Fig. 9). Erkennbar ist, dass die Fluidweiche 6 bei dieser Ausführungsform als ein im Wesentlichen zylinderförmiges Bauteil ausgebildet ist, das in einem Mantelflächenabschnitt eine Abflachung 27 aufweist, die zum einen am fertigen Griffelement 4 (siehe insbesondere Fig. 3 und 4) einen Griffbereich für einen Operateur, zum anderen eine zuverlässige Fixierung des Fluidweichenelementes 6 innerhalb des Außenmantels 11 (siehe Fig. 2 und 3) bildet.

An seiner einen Stirnseite 7 weist das Fluidweichenelement 6 den Applikationsleitungsanschlussbereich 32 auf, der der Aufnahme der Applikationssonde 2 und der darin ausgebildeten Applikationsleitungselemente 13, 23 (siehe Fig. 2 und 3) dient.

An seiner anderen Stirnseite 9 weist das Fluidweichenelement 6 den Versorgungsleitungsanschlussbereich 33 auf, der dem Anschluss der beiden Versorgungsleitungselemente 13, 23 des Schlauchsatzes 3 (siehe Fig. 2 und 3) dient.

Der Versorgungsleitungsanschlussbereich 33 für jedes anzuschließende Versorgungsleitungselement 13, 23 weist jeweils ein komplementäres Versorgungsleitungsanschlusselement 14, 24 auf, über das das jeweilige Versorgungsleitungselement 13, 23 (siehe Fig. 2 und 3) mit dem korrespondierenden Überleitungselement 16, 26 (siehe insbesondere Fig. 7) fluiddicht verbindbar ist, wobei die Versorgungsleitungsanschlusselemente 14, 24 und die Versorgungsleitungselemente 13, 23 bei dieser Ausführungsform derart komplementär zueinander ausgebildet sind, dass wahlweise jedes Versorgungsleitungselement 13, 23 an jedes Versorgungsleitungsanschlusselement 14, 24 anschließbar ist.

Darüber hinaus ist es bei dieser Ausführungsform möglich, das erfindungsgemäße Griffelement mit unterschiedlichen "Beschickungsrichtungen" zu betreiben, da es über den Filteraufnahmebereich 28 möglich ist, das Filterelement 29 (siehe Fig. 2 und 3) so anzuordnen, dass das Filterelement immer im zuführenden Leitungssystem angeordnet ist. Auf diese Weise lässt sich also ein Kryochirurgiegerät gemäß den Ausführungsformen aus Fig. 2 und 3 entweder im "normalen" Kryochirurgiebetrieb oder im Defreeze-Betrieb betreiben, da es möglich ist, das Versorgungsleitungselement 13 bzw. 23 entweder als Zu- oder als Ableitungselement zu verwenden.

### Bezugszeichenliste

- 1: Chirurgieinstrument
- 2: Applikationssonde
- 3: Versorgungsschlauchsatz
- 4: Griffelement
- 5: Handgriff
- 6: Fluidweichenelement
- 7: Stirnseite
- 8: proximales Ende
- 9: Stirnseite
- 11: Außenmantel
- 12: Applikationsleitungselement
- 13: Versorgungsleitungselement
- 14: Versorgungsleitungsanschlusselement
- 15: Überleitungselement
- 17: Aufnahmebereich
- 19: Aufnahmebereich
- 21: Aufnahmeformteil
- 22: Applikationsleitungselement
- 23: Versorgungsleitungselement
- 24: Versorgungsleitungsanschlusselement
- 25: Arretierungsmittel
- 26: Überleitungselement
- 27: Abflachung
- 28: Filteraufnahmeraum
- 29: Filterelement
- 30: Anschlussvorrichtung
- 32: Applikationsleitungsanschlussbereich
- 33: Versorgungsleitungsanschlussbereich
- 40: Versorgungseinheit

## Patentansprüche

1. Handgriff für ein Chirurgieinstrument mit
einem Griffelement (4),
einem Versorgungsschlauchsatz (3), mit wenigstens zwei parallel geführten Versorgungsleitungselementen (13, 23), wenigstens einer Applikationssonde (2), mit wenigstens zwei koaxial geführten Applikationsleitungselementen (12, 22),
wobei jedes Verso-rgungsleitungselement (13, 23) im Griffelement (4) mit einem zugehörigen Applikationsleitungselement (12, 22) verbindbar ist, **gekennzeichnet durch**
ein Fluidweichenelement (6) zum fluidführenden Anschluss der wenigstens einen Applikationssonde (2) an den Versorgungsschlauchsatz (3),
wobei das Fluidweichenelement (6) als eigenständiges Bauteil in dem Griffelement (4) des chirurgischen Instrumentes (1) zum fluidführenden Anschluss der Applikationssonde (2) an den Versorgungsschlauchsatz (3) angeordnet ist,
wobei im angeschlossenen Zustand jedes Versorgungsleitungselement (13, 23) mit wenigstens einem zugehörigen Applikationsleitungselement (12, 22) in Fluidverbindung steht,
wobei das Fluidweichenelement (6) weiter aufweist:
- einen Applikationsleitungsanschlussbereich (32) mit einem integral ausgebildeten Aufnahmebereich (17), zum koaxialen Anschluss und fluiddichten Verbindung der Applikationsleitungselemente (12, 22) innerhalb des Aufnahmebereichs (17) mit dem Fluidweichenelement (6),
- einen Versorgungsleitungsanschlussbereich (33) mit einem integral ausgebildeten Aufnahmebereich (19), zum Anschluss und fluiddichten Verbindung der Versorgungsleitungselemente (13, 23) innerhalb des Aufnahmebereichs (19) mit dem Fluidweichenelement (6), und
- wenigstens zwei integral in dem Fluidweichenelement (6) ausgebildete Überleitungselemente (16, 26), über die der Applikationsleitungsanschlussbereich (32) mit dem Versorgungsleitungsanschlussbereich (33) in Fluidverbindung steht, wobei
- der Versorgungsleitungsanschlussbereich (33) und der Applikationsleitungsanschlussbereich (32) in dem integral in dem Fluidweichenelement (6) ausgebildeten Aufnahmebereich (17, 19) derart angeordnet sind, dass sie nicht über die Außengeometrie des Fluidweichenelements (6) hervorstehen, und
- einen Applikationssondenaufnahmebereich (20) zur lösbaren Aufnahme der Applikationssonde (2), der wenigstens ein Arretierungsmittel (25) aufweist, über das die Applikationssonde (2) werkzeuglos lösbar am Fluidweichenelement (6) arretierbar istwobei das Fluidweichenelement als ein einstückiges Drehteil oder Frästeil ausgebildet ist.

2. Handgriff nach einem der vorhergehenden Ansprüche,
**dadurch gekennzeichnet, dass**
es wenigstens teilweise als langgestreckter Körper ausgebildet ist.

3. Handgriff nach Anspruch 2,
**dadurch gekennzeichnet, dass**
der langgestreckte Körper als Zylinderelement ausgebildet ist.

4. Handgriff nach Anspruch 2 oder 3,
**dadurch gekennzeichnet, dass**
der Versorgungsleitungsanschlussbereich und/oder der Applikationsleitungsanschlussbereich (32) an Stirnseiten (7, 9) des langgestreckten Volumenkörpers angeordnet sind,

5. Handgriff nach einem der vorhergehenden Ansprüche,
**dadurch gekennzelchnet, dass**
der Versorgungsleitungsanschlussbereich (33) für jedes anzuschließende Versorgungsleitungselement (13, 23) jeweils ein komplementäres Versorgungsleitungsanschlusselement (14, 24) aufweist, über das das jeweilige Versorgungsleitungselement (13, 23) mit dem korrespondierenden Überleitungselement (16, 26) fluiddicht verbindbar ist, wobei die Versorgungsleitungsanschlusselemente (14, 24) und/oder die Versorgungsleitungselemente (13, 23) derart komplementär zueinander ausgebildet sind, das wahlweise jedes Versorgungsleitungselement (13, 23) an jedes Versorgungsleitungsanschlusselement (14, 24) anschließbar ist.

6. Handgriff nach einem der vorhergehenden Ansprüche,
**dadurch gekennzeichnet, dass**
im Fluidweichenelement (6) Integral mindestens ein Filteraufnahmeraum (28) zur Aufnahme eines Filterelementes (29) derart ausgebildet ist, dass das Filterelement (29) innerhalb wenigstens eines Fluidweges zwischen einem Versorgungsleitungselement (13, 23) und einem Applikationsleitungselement (12, 22), Im zuführenden Fluidweg, angeordnet ist.

## Claims

1. Handle for a surgical instrument with
a handle element (4),
a supply tubing set (3) with at least two parallel guided supply line elements (13, 23), at least one application probe (2) with at least two coaxially guided application line elements (12, 22),
wherein each supply line element (13, 23) in the handle element (4) can be connected to as associated application line element (12, 22),
and a fluid-switch element (6) for fluid-conductive attachment of the at least one application probe (2) to the supply tubing set (3),
wherein the fluid-switch element (6) is arranged as an autonomous component in the handle element (4) of the surgical instrument (1) for fluid-conductive attachment of the application probe (2) to the supply tubing set (3),
wherein in the attached state, each supply line element (13, 23) is in fluidic connection with at least one associated application line element (12, 22),
wherein the fluid-switch element (6) furthermore comprises:
- an application line attachment region (32) with an integrally formed receiving region (17) for coaxial attachment and fluid-tight connection of the application line elements (12, 22) inside the receiving region (17) to the fluid-switch element (6),
- a supply line attachment region (33) with an integrally formed receiving region (19) for attachment and fluid-tight connection of the supply line elements (13, 23) inside the receiving region (19) to the fluid-switch element (6), and
- at least two bridging elements (16, 26) which are formed integrally in the fluid-switch element (6) and via which the application line attachment region (32) is in fluidic connection with the supply line attachment region (33), wherein the supply line attachment region (33) and the application line attachment region (32) are arranged in the receiving region (17, 19) formed integrally in the fluid-switch element (6) such that they do not protrude beyond the outer geometry of the fluid-switch element (6), and
- an application probe receiving region (20) for releasable receiving of the application probe (2), which has at least one locking means (25) via which the application probe (2) can be locked releasably to the fluid-switch element (6) without tools,
- wherein the fluid-switch element is configured as a one-piece turned or milled part.

2. Handle according to any of the preceding claims, **characterised in that** the fluid-switch element (6) is formed at least partially as an elongate body.

3. Handle according to claim 2, **characterised in that** the elongate body is configured as a cylinder element.

4. Handle according to claim 2 or 3, **characterised in that** the supply line attachment region and/or the application line attachment region (32) are arranged on end faces (7, 9) of the elongate volume body.

5. Handle according to any of the preceding claims, **characterised in that** the supply line attachment region (33) has, for each supply line element (13, 23) to be connected, a complementary supply line connecting element (14, 24) via which the respective supply line element (13, 23) can be connected fluid-tightly to the corresponding bridging element (16, 26), wherein the supply line attachment elements (14, 24) and/or the supply line elements (13, 23) are configured complementary to each other such that optionally each supply line element (13, 23) can be attached to each supply line attachment element (14, 24).

6. Handle according to any of the preceding claims, **characterised in that** at least one filter receiving chamber (28) for receiving a filter element (29) is formed integrally in the fluid-switch element (6) such that the filter element (29) is arranged inside at least one fluid path between a supply line element (13, 23) and an application line element (12, 22) in the supplying fluid path.

## Revendications

1. Poignée destinée à un instrument chirurgical, comprenant un élément de poignée (4),
un ensemble de tuyaux flexibles d'alimentation (3), comportant au moins deux éléments de conduite d'alimentation (13, 23) guidés de façon parallèle,
au moins une sonde d'application (2), comportant au moins deux éléments de conduite d'application (12, 22) guidés de façon coaxiale,
sachant que chaque élément de conduite d'alimentation (13, 23) peut être relié, dans l'élément de poignée (4), à un élément de conduite d'application (12, 22) associé,
et comprenant un élément d'aiguillage de fluide (6) destiné au raccordement transportant des fluides de la sonde d'application (2), au nombre d'au moins une, à l'ensemble de tuyaux flexibles d'alimentation (3)
sachant que l'élément d'aiguillage de fluide (6) est disposé en tant que composant autonome dans l'élément de poignée (4) de l'instrument chirurgical (1), en vue du raccordement transportant des fluides de la sonde d'application (2), à l'ensemble de tuyaux flexibles d'alimentation (3),
sachant qu'à l'état raccordé, chaque élément de conduite d'alimentation (13, 23) est en communication de fluide avec au moins un élément de conduite d'application (12, 22) associé :
l'élément d'aiguillage de fluide (6) présentant en outre :
- une zone de raccordement de conduite d'application (32) avec une zone de réception (17) formée d'une seule pièce, en vue du raccordement coaxial et de la liaison étanche aux fluides des éléments de conduite d'application (12, 22) à l'élément d'aiguillage de fluide (6), à l'intérieur de la zone de réception (17),
- une zone de raccordement de conduite d'alimentation (33) avec une zone de réception (19) formée d'une seule pièce, en vue du raccordement et de la liaison étanche aux fluides des éléments de conduite d'alimentation (13, 23) à l'élément d'aiguillage de fluide (6), à l'intérieur de la zone de réception (19), et
- au moins deux éléments de transition (16, 26) qui sont formés d'une seule pièce dans l'élément d'aiguillage de fluide (6) et par l'intermédiaire desquels la zone de raccordement de conduite d'application (32) est en communication de fluide avec la zone de raccordement de conduite d'alimentation (33), sachant que
la zone de raccordement de conduite d'alimentation (33) et la zone de raccordement de conduite d'application (32) sont disposées dans la zone de réception (17, 19), formée d'une seule pièce dans l'élément d'aiguillage de fluide (6), de manière à ce qu'elles ne dépassent pas par rapport à la géométrie extérieure de l'élément d'aiguillage de fluide (6), et
il y a une zone de réception de sonde d'application (20) qui est destinée à la réception amovible de la sonde d'application (2) et présente au moins un moyen de verrouillage (25) par l'intermédiaire duquel la sonde d'application (2) peut être verrouillée de façon amovible, sans outil, à l'élément d'aiguillage de fluide (6), sachant que
l'élément d'aiguillage de fluide (6) est réalisé comme pièce tournée ou pièce fraisée monobloc.

2. Poignée selon l'une des revendications précédentes, **caractérisée en ce que** l'élément d'aiguillage de fluide (6) est réalisé au moins en partie comme corps allongé.

3. Poignée selon la revendication 2, **caractérisée en ce que** le corps allongé est réalisé comme élément cylindrique.

4. Poignée selon la revendication 2 ou 3, **caractérisée en ce que** la zone de raccordement de conduite d'alimentation et/ou la zone de raccordement de conduite d'application (32) est/sont disposée(s) sur des faces frontales (7, 9) du corps volumique allongé.

5. Poignée selon l'une des revendications précédentes, **caractérisée en ce que** la zone de raccordement de conduite d'alimentation (33) présente pour chaque élément de conduite d'alimentation (13, 23) à raccorder, respectivement un élément de raccordement de conduite d'alimentation (14, 24) complémentaire, par l'intermédiaire duquel l'élément de conduite d'alimentation (13, 23) respectif peut être relié de manière étanche aux fluides à l'élément de transition (16, 26) correspondant, sachant que les éléments de raccordement de conduite d'alimentation (14, 24) et/ou les éléments de conduite d'alimentation (13, 23) sont réalisés de façon complémentaire les uns par rapport aux autres telle qu'au choix, chaque élément de conduite d'alimentation (13, 23) peut être raccordé à chaque élément de raccordement de conduite d'alimentation (14, 24).

6. Poignée selon l'une des revendications précédentes, **caractérisée en ce que** dans l'élément d'aiguillage de fluide (6), au moins un logement de filtre (28) est réalisé d'une seule pièce en vue de recevoir un élément de filtre (29), de façon telle que l'élément de filtre (29) est disposé à l'intérieur d'au moins une voie pour fluide, entre un élément de conduite d'alimentation (13, 23) et un élément de conduite d'application (12, 22), dans la voie d'amenée de fluide.
